# EUROPEAN PATENT APPLICATION

(11) **EP 4 786 985 A1**
(43) Date of publication of application: **05.08.2026**
(21) Application number: 24872241.5
(22) Date of filing: 25.09.2024
(51) Int. Cl.: G01N 33/68, G01N 21/78, G01N 33/52

(54) **TEST PIECE FOR PROTEIN MEASUREMENT AND PROTEIN MEASUREMENT METHOD**

(30) Priority: 26.09.2023 JP 2023163990
(71) Applicant: FUJIFILM Corporation, Tokyo 106-8620 (JP)
(72) Inventor: YOSHIDA, Junya, Kanagawa 2588538 (JP); MORIGUCHI, Yusuke, Kanagawa 2588538 (JP); YAMAGUCHI, Yukihiro, Kanagawa 2588538 (JP)
(74) Representative: Dehns Germany Partnerschaft mbB
(86) International application number: PCT/JP2024/034082
(87) International publication number: WO 2025/070455

(57) **Abstract**

An object of the present invention is to provide a test piece for measuring protein, in which the detection sensitivity during the measurement is improved, and an optical density that can measure the protein in the sample even in a case where a large amount of protein is contained in the sample can be obtained; and a method for measuring protein using the test piece for measuring protein. According to the present invention, there is provided a test piece for measuring protein, including a support and a reagent-holding layer provided on the support, in which the reagent-holding layer contains a coloring agent that changes color in a presence of protein, a molybdate, and a hydroxy acid having a hydroxy group and a carboxy group, and a content of the hydroxy acid is 5 times or more and 40 times or less with respect to the coloring agent that changes color in the presence of protein in terms of molar ratio.

## Description

### Technical Field

The present invention relates to a test piece for measuring protein and a method for measuring protein, using a coloring agent that changes color in the presence of protein.

### Background Art

A test for quantifying protein contained in a sample derived from various organisms including humans is required to be accurate and highly sensitive in order to perform diagnosis and treatment of various diseases. As one of test methods for quantifying protein, a method using pyrocatechol violet (also referred to as PV) and ammonium molybdate is known. The complex formed from pyrocatechol violet and ammonium molybdate has a characteristic that an absorption wavelength changes in an amount-dependent manner as a result of interaction with protein. A test piece for detecting and quantifying protein using this characteristic is known.

Patent Document 1 describes a test piece for detecting protein in urine, the test piece including an absorbent carrier impregnated with a reagent system containing a buffer and a protein error indicator that changes color in the presence of protein, in which tartaric acid as a cation-sensitive agent is used, in combination with a non-cation-sensitive buffer, in an amount that can prevent the pH of the reagent from increasing to a level at which the protein error indicator changes color even in a case where a significant amount of protein is not present in the urine.

Patent Document 2 describes a test piece for measuring protein, the test piece consisting of an absorbent carrier containing a composition for measuring protein, the composition consisting of (1) a molybdate, (2) a coloring agent, (3) a chelating agent that binds to molybdenum or a metal ion that does not react with the above-described coloring agent and is capable of binding to oxalic acid, citric acid, or the like, and (4) a buffer.

Patent Document 3 describes a dry analytical element used for measuring protein, including: (A) a porous spreading layer; (B) one or more additional layers containing (i) a coloring agent, (ii) a molybdate, and (iii) a polymer containing acrylamide; and (C) a support.

### Prior Art Documents

### Patent Documents

Patent Document 1: JP3621209B
Patent Document 2: JP-S62-006170A
Patent Document 3: JP-H11-337547A

### Summary of Invention

### Object to be solved by the invention

In the test pieces described in Patent Documents 1 to 3, a hydroxy acid having a hydroxy group and a carboxy group is used to prevent the coloring agent such as PV from changing color in a case of measuring a sample in which protein is not present. However, in a case where ΔOD, which is a difference between an OD (magnitude of a signal) in a case where protein is present and an OD in a case where protein is not present, is small, there is an issue that the detection sensitivity is reduced. In addition, it is not possible to obtain an optical density (degree of color change) sufficient to allow measurement of protein in the sample not only at a concentration at a boundary between normal and abnormal, but also in a case where a large amount of protein is contained in the sample. Therefore, the measurement sensitivity is not sufficient.

An object to be achieved by the present invention is to provide a test piece for measuring protein, in which the detection sensitivity during the measurement is improved, and an optical density that can measure the protein in the sample even in a case where a large amount of protein is contained in the sample can be obtained. Another object to be achieved by the present invention is to provide a method for measuring protein using the test piece for measuring protein.

### Means for solving the object

As a result of intensive studies to achieve the above object, the present inventors have found that, in the test piece for measuring protein, having a reagent-holding layer containing a coloring agent that changes color in the presence of protein, a molybdate, and a hydroxy acid, by setting a content of the hydroxy acid to 5 times or more and 40 times or less with respect to the coloring agent that changes color in the presence of protein in terms of molar ratio, the hydroxy acid interacts with the complex of PV and ammonium molybdate in a case where the protein is not present in the sample, and the complex formation is hindered, thereby reducing the absorbance of the complex, and at the same time, a sufficient concentration is obtained in a case where the protein is present in the sample. The present invention has been completed based on the above findings. According to the present invention, the following inventions are provided.
<1> A test piece for measuring protein, comprising: a support; and a reagent-holding layer provided on the support, in which the reagent-holding layer contains a coloring agent that changes color in a presence of protein, a molybdate, and a hydroxy acid having a hydroxy group and a carboxy group, and a content of the hydroxy acid is 5 times or more and 40 times or less with respect to the coloring agent that changes color in the presence of protein in terms of molar ratio.
<2> The test piece for measuring protein according to <1>, in which the coloring agent that changes color in the presence of protein is pyrogallol red or pyrocatechol violet.
<3> The test piece for measuring protein according to <1>, in which the coloring agent that changes color in the presence of protein is pyrocatechol violet.
<4> The test piece for measuring protein according to any one of <1> to <3>, in which the hydroxy acid is a compound having two or more carboxy groups.
<5> The test piece for measuring protein according to any one of <1> to <4>, in which the hydroxy acid includes at least one of tartaric acid, citric acid, or malic acid.
<6> The test piece for measuring protein according to any one of <1> to <5>, in which the reagent-holding layer includes a water-absorbent layer and a spreading layer.
<7> A method for measuring protein, comprising: spotting a protein-containing sample on the test piece for measuring protein according to any one of <1> to <6>; and measuring color development in the reagent-holding layer.

### Effect of the invention

According to the test piece for measuring protein and the method for measuring protein according to the present invention, the detection sensitivity during the measurement is improved, and an optical density that can measure the protein in the sample even in a case where a large amount of protein is contained in the sample can be obtained.

### Embodiments for carrying out the invention

Hereinafter, the present invention is specifically described.

In the present specification, "to" shows a range including numerical values described before and after "to" as a minimum value and a maximum value.

In the present invention, a test piece for measuring protein is provided, the test piece including a support and a reagent-holding layer provided on the support, in which the reagent-holding layer contains a coloring agent that changes color in the presence of protein, a molybdate, and a hydroxy acid having a hydroxy group and a carboxy group, and a content of the hydroxy acid is 5 times or more and 40 times or less with respect to the coloring agent that changes color in the presence of protein in terms of molar ratio.

In the present invention, it has been found that, in the test piece including the coloring agent that changes color in the presence of protein and the molybdate, by including the hydroxy acid that is 5 times or more and 40 times or less with respect to the coloring agent that changes color in the presence of protein in terms of molar ratio, the signal before the protein detection (hereinafter, background) is reduced while the signal after the protein detection (hereinafter, signal) is maintained, and ΔOD (calculated by signal - background) can be improved.

The amount of protein contained in a biological specimen, particularly in urine, is very useful as an indicator for simply evaluating the presence or absence of kidney dysfunction, and the presence or absence of a tumor or inflammation of a bladder, a ureter, or a prostate. The kidney adjusts the amount of protein in the body, and unnecessary protein is filtered by the kidney and excreted into the urine. However, in a case where the kidney function is reduced, the protein necessary for the body is also excreted from the kidney, and the amount of protein in the urine increases. By detecting this change, it is possible to detect the disease as described above. The type of the protein is not particularly limited, and examples thereof include albumin.

The test piece according to the present invention is a test piece for measuring protein, the test piece including at least a support and a reagent-holding layer provided on the support, and the reagent-holding layer may be one layer or may be divided into a plurality of layers. It is preferable that at least one layer of the reagent-holding layers contains a coloring agent that changes color in the presence of protein, a molybdate, and a hydroxy acid having a hydroxy group and a carboxy group.

### (Coloring agent that changes color in presence of protein)

As the coloring agent that changes color in the presence of protein, pyrogallol red or pyrocatechol violet is preferable, and pyrocatechol violet is more preferable.

The amount of the coloring agent used in the reagent-holding layer is preferably 0.005 g/m² to 0.5 g/m², and more preferably 0.02 g/m² to 0.2 g/m².

### (Molybdate)

As the molybdate, ammonium molybdate, sodium molybdate, potassium molybdate, lithium molybdate, calcium molybdate, magnesium molybdate, or the like is preferably used, and ammonium molybdate can be more preferably used.

The content of ammonium molybdate in the reagent-holding layer is preferably 0.008 g/m² to 0.8 g/m², and more preferably 0.05 to 0.5 g/m².

### (Hydroxy acid having hydroxy group and carboxy group)

As the hydroxy acid having a hydroxy group and a carboxy group, a hydroxy acid having a hydroxy group at an α-position of a carboxy group is preferably used. As the hydroxy acid having a hydroxy group and a carboxy group, a compound having two or more carboxy groups is also preferable.

As the hydroxy acid having a hydroxy group and a carboxy group, tartaric acid, malic acid, citric acid, glycolic acid, 2-hydroxybutyric acid, isocitric acid, or lactic acid can be more preferably used, and at least one of tartaric acid, citric acid, or malic acid can be still more preferably used.

The amount of the hydroxy acid used in the reagent-holding layer is preferably 0.025 g/m² to 2.5 g/m², and more preferably 0.1 g/m² to 1.0 g/m². Provided that as the amount of the hydroxy acid used, an amount that satisfies a ratio of the amount of the hydroxy acid to the coloring agent described below is used.

### (Mass ratio of amount of hydroxy acid to coloring agent)

As a result of intensive studies, in the present invention, it has been found that, in a case where the protein is not present in the sample, the complex formation between the coloring agent that changes color in the presence of protein and the molybdate is hindered, thereby reducing the absorbance of the complex, and at the same time, in a case where the protein is present in the sample, an effect is obtained in a case where the molar ratio of the amount of the hydroxy acid to the coloring agent in the reagent-holding layer is in a range of 5 times or more and 40 times or less, such that a sufficient absorbance is obtained. Furthermore, a molar ratio of the amount of the hydroxy acid to the coloring agent in the reagent-holding layer at which the effect of the present invention can be sufficiently obtained, is preferably 10 times or more and 40 times or less, more preferably 12 times or more and 36 times or less, and still more preferably 12 times or more and 20 times or less. In a case where the ratio of the hydroxy acid to the coloring agent is less than 5 times, it is expected that the complex formation inhibitory effect on the coloring agent and ammonium molybdate is insufficient, and the absorbance reduction in a case where the protein is not present is insufficient. On the other hand, in a case where the ratio of the hydroxy acid to the coloring agent is more than 40 times, it is expected that the complex of the coloring agent and ammonium molybdate is not sufficiently formed even in a case where the protein is present, and the absorbance is not sufficiently obtained.

The test piece for measuring protein according to the embodiment of the present invention includes a support and a reagent-holding layer provided on the support.

The reagent-holding layer may include a water-absorbent layer and a spreading layer.

The spreading layer is a layer having an action (metering action) of spreading an aqueous liquid sample, which is supplied in spots onto the upper surface of the test piece for measuring a protein, in a lateral direction without substantially unevenly distributing the components contained in the aqueous liquid sample, and supplying the aqueous liquid sample to a water-absorbent layer containing a water-soluble polymer having a water absorbency at a ratio of a substantially constant capacity per unit area.

In addition, a layer such as an adhesive layer can also be provided between the support and the layer provided thereon and between the respective layers provided on the support.

In addition, the above-described water-absorbent layer and spreading layer may be provided as separate layers, but may also be provided as one layer by allowing the same layer to have the above-described two or more functions at the same time. That is, the water-absorbent layer and the spreading layer may be provided as the same layer.

As the support, a water-impermeable support is preferable. As the material of the water-impermeable support, a polymer such as polyethylene terephthalate, polycarbonate of bisphenol A, polystyrene, or cellulose ester (for example, cellulose diacetate, cellulose triacetate, cellulose acetate propionate, or the like) is preferable, and polyethylene terephthalate is particularly preferable. As the support, a smooth planar support that is transparent, for example, transmits electromagnetic radiation having a wavelength in a range of at least a part of a wavelength range of approximately 200 nm to approximately 900 nm, in a thickness range of approximately 50 µm to approximately 1 mm and preferably approximately 80 µm to approximately 300 µm can be used. A known undercoat or adhesive layer can be provided on the surface of the support to strengthen the adhesion to the water-absorbent layer.

The water-absorbent layer is preferably a layer containing a water-soluble polymer that can swell and absorb water by being brought into contact with water. The water-absorbent layer has an action of improving the development of the aqueous liquid sample in the spreading layer in a case where the water in the aqueous liquid sample spotted on the spreading layer reaches the upper surface of the water-absorbent layer during the analysis operation.

The content of the water-soluble polymer in the water-absorbent layer is preferably 10 to 100 g/m² and more preferably 20 to 70 g/m².

The water-soluble polymer used in the water-absorbent layer is preferably a non-proteinaceous water-soluble polymer having a property of swelling and absorbing water in a case of being in contact with water. Examples of the non-proteinaceous water-soluble polymer include polyacrylamide; agarose; acrylamide-based copolymers such as acrylamide-N-vinylpyrrolidone copolymer described in JP1982-50660A (JP-S57-50660A), JP1983-77664A (JP-S58-77664A), and the like; polyvinyl alcohol; methallyl alcohol-based copolymers such as a binary or ternary copolymer of methallyl alcohol with acrylamide or a derivative thereof, acrylic acid or a derivative thereof, methacrylic acid or a derivative thereof, or N-vinyl-2-pyrrolidone, described in JP1987-137564A (JP-S62-137564A) (the methallyl alcohol-based copolymer is crosslinkable, for example, an acrylamide-N-vinyl-2-pyrrolidone-methallyl alcohol ternary copolymer); and the like.

Among these non-proteinaceous water-soluble polymers, an acrylic amide-based copolymer such as an acrylamide-N-vinylpyrrolidone copolymer or a methallyl alcohol-containing copolymer such as an acrylamide-N-vinyl-2-pyrrolidone-methallyl alcohol ternary copolymer is preferable.

The water-absorbent layer can contain various components that do not adversely affect the capability of the coloring agent. An example thereof is a nonionic surfactant. Specific examples of the nonionic surfactant include the same ones as the surfactant that can be contained in the reagent-holding layer. By containing the nonionic surfactant in the water-absorbent layer, water in the aqueous liquid sample is likely to be substantially uniformly absorbed into the water-absorbent layer during the analysis operation, and the liquid contact with the spreading layer is rapidly and substantially uniform.

The water-absorbent layer can contain a crosslinking agent (also referred to as a curing agent or a hardening agent). As the crosslinking agent, various inorganic and organic crosslinking agents well known in the field of organic polymer chemistry can be used. Examples of the organic crosslinking agent for polyvinyl alcohol include dimethyl urea, and examples of the organic crosslinking agent for a methallyl alcohol-containing polymer include formaldehyde. The content of the crosslinking agent in the water-absorbent layer can be selected depending on the coating amount and the degree of curing of the water-absorbent layer to be crosslinked and cured, but is generally in a range of approximately 50 mg/m² to approximately 5,000 mg/m² and preferably in a range of approximately 100 mg/m² to approximately 2,000 mg/m² in terms of the coating amount.

As the spread layer, for example, a woven spreading layer (for example, a plain woven fabric such as broadcloth or poplin) described in JP1980-164356A (JP-S55-164356A), JP1982-66359A (JP-S57-66359A), or the like; a knitted spreading layer (for example, a tricot knitted fabric, a double tricot knitted fabric, a Milanese knitted fabric, or the like) described in JP1985-222769A (JP-S60-222769A) or the like; a spreading layer consisting of wet-laid paper containing organic polymer fiber pulp as described in JP1982-148250A (JP-S57-148250A); a nonfibrous isotropic porous spreading layer such as a membrane filter (brush polymer layer) as described in JP1978-21677A (JP-S53-21677A) and US3992158A, or a continuous microporous porous layer in which polymer microbeads, glass microbeads, or diatomaceous earth are held by a water-soluble polymer binder; a nonfibrous isotropic porous spreading layer composed of a continuous microporous porous layer (three-dimensional lattice-like particulate structure layer) in which polymer microbeads are bond in point contact with each other by a polymer adhesive that does not swell or expand in water as described in JP1980-90859A (JP-S55-90859A); or the like can be used. As the spreading layer, a knitted spreading layer (for example, a tricot knitted fabric, a double tricot knitted fabric, a Milanese knitted fabric, or the like) is preferable.

The spreading layer contains a coloring agent that changes color in the presence of protein, a molybdate, and a hydroxy acid having a hydroxy group and a carboxy group.

Examples of the water-soluble polymer that can be included in the spreading layer include acrylic polymers (such as polyacrylamide), cellulose-based polymers (such as carboxymethyl cellulose and hydroxymethyl cellulose), and the like. As the water-soluble polymer, polyvinyl alcohol, polyvinyl ether, polyvinylpyrrolidone, or the like can be preferably used.

A coating amount of the water-soluble polymer in the spreading layer is preferably 0.1 g/m² to 30 g/m², more preferably 0.3 g/m² to 20 g/m², and still more preferably 0.5 g/m² to 15 g/m².

The pH of a liquid of the spreading layer is preferably 1.0 to 5.0, more preferably 1.5 to 4.5, and still more preferably 2.0 to 4.0. The pH of a liquid of the spreading layer means the pH of the coating liquid used for forming the spreading layer.

Examples of the method for including the reagent in the spreading layer include a method of substantially uniformly applying or spraying an aqueous solution, a water-organic solvent mixed solvent solution, or an organic solvent solution (examples of the organic solvent: aliphatic alcohols such as methanol, ethanol, and isopropyl alcohol; dialkyl ketones such as acetone and methyl ethyl ketone; dialkyl ethers such as dimethyl ether; aliphatic cyclic ethers such as tetrahydrofuran and dioxane; acetonitrile; hexane; β-methoxyethanol; ethylene glycol; and the like) containing a coloring agent that changes color in the presence of protein, a molybdate, and a hydroxy acid having a hydroxy group and a carboxy group, from above the spreading layer and drying by a known method; a method of immersing a material for a spreading layer in a solution containing a coloring agent that changes color in the presence of protein, a molybdate, and a hydroxy acid having a hydroxy group and a carboxy group and laminating the material on an water-absorbent layer (water-soluble polymer binder layer) in a dry or semi-dry state to integrate the material; and the like.

The test piece for measuring protein according to the embodiment of the present invention can be prepared by a method known to those skilled in the art. For example, a coating liquid prepared as a water-absorbent layer coating liquid is applied onto the support and dried to produce a dried film having a thickness of about 40 µm, and then a woven fabric of the spreading layer is bonded thereto. Thereafter, the coating liquid prepared as the reagent-holding layer liquid is applied from the woven fabric side of the spreading layer and dried, whereby a test piece for measuring protein can be prepared. It is preferable that the test piece for measuring protein is cut into small pieces such as a square having one side of approximately 15 mm to approximately 30 mm or substantially the same size as the square, and used as a chemical analysis slide by being housed in a slide frame described in JP1982-28331B (JP-S57-28331B), JP1981-142454B (JP-S56-142454B), JP1982-63452A (JP-S57-63452A), JP1983-32350A (JP-S58-32350A), JP1983-501144A (JP-S58-501144A), from various viewpoints such as manufacturing, packaging, transportation, storage, and measurement operations. Depending on the intended use, the test piece can be used in a long tape shape by being housed in a cassette or a magazine, or can be used in a small piece shape by being attached to or housed in a card having an opening.

According to the present invention, a method for measuring protein is provided, the method including spotting a protein-containing sample on the test piece for measuring protein according to the embodiment of the present invention; and measuring color development in the reagent-holding layer.

For example, the content of protein in the liquid sample can be determined by spotting an aqueous liquid sample such as whole blood, blood plasma, serum, lymph fluid, or urine, in a range of approximately 5 µL to approximately 30 µL and preferably approximately 8 µL to approximately 15 µL, on the spreading layer, incubating the aqueous liquid sample at a substantially constant temperature in a range of approximately 20°C to approximately 40°C and preferably at a substantially constant temperature in the vicinity of 37°C for a range of approximately 1 minute to approximately 10 minutes and preferably approximately 2 minutes to approximately 7 minutes, and measured, by reflection photometry, the detectable change such as the color change or the color development in the test piece for measuring protein from the support side, according to the principle of the colorimetry. In the present invention, the optical density of the spreading layer is measured by reflection photometry using light having an absorption maximum wavelength of protein-coloring agent binding or a wavelength in the vicinity thereof, and the protein content in the liquid sample can be determined according to the principle of the colorimetry using a calibration curve created in advance. By keeping the amount of the aqueous liquid sample to be spotted, the incubation time, and the incubation temperature constant, the quantitative analysis of the protein can be performed with high accuracy. In the measurement operation, it is possible to perform a highly accurate quantitative analysis by a chemical analysis apparatus described in JP1985-125543A (JP-S60-125543A), JP1985-220862A (JP-S60-220862A), JP1986-294367A (JP-S61-294367A), JP1983-161867A (JP-S58-161867A), and the like in an extremely easy operation.

Next, the present invention will be described using Examples, but the present invention is not limited thereto.

### Examples

### <Example 1>

Each of the components was applied to a polyethylene terephthalate colorless transparent smooth film in the following amounts and dried.

### (Undercoat layer)

| | |
|---|---|
| Polyvinyl alcohol 5-74 (manufactured by Kuraray Co., Ltd.) | 23.14 g/m² |
| ARALDITE DY022 (manufactured by Nagase ChemteX Corporation) | 0.78 g/m² |

The pH of the aqueous solution was set to 3.05.

Next, water was supplied to the entire surface of the above-described film in a supply amount of approximately 12 mg/(10mm)² to wet the film, and then a tricot knitted fabric obtained by knitting a polyethylene terephthalate spun yarn corresponding to 50 denier at 36 gauge was laminated with light pressure, and dried.

Next, an aqueous solution (pH 2.5) having the following composition was applied such that each component had the following amount, and dried to produce an integrated multilayer analytical element.

### (Spreading layer)

| | |
|---|---|
| Tartaric acid (manufactured by FUJIFILM Wako Pure Chemical Corporation) | 0.267 g/m² |
| PV (manufactured by Tokyo Chemical Industry Co., Ltd.) | 0.114 g/m² |
| Ammonium molybdate (manufactured by FUJIFILM Wako Pure Chemical Corporation) | 0.172 g/m² |
| Hydroxypropyl methylcellulose (manufactured by Shin-Etsu Chemical Co., Ltd.) | 2.55 g/m² |
| Modified silicone oil (Product name KF353, manufactured by Shin-Etsu Chemical Co., Ltd.) | 0.86 g/m² |
| Succinic acid (manufactured by FUJIFILM Wako Pure Chemical Corporation) | 5.62 g/m² |

Here, tartaric acid was applied such that the molar ratio to PV was 6.03.

The above-described integrated multilayer analytical element was cut into a chip having a square of 12 mm × 13 mm, and housed in a slide frame (described in JP1982-63452A (JP-S57-63452A)) to produce a test piece 6 for measuring protein according to the embodiment of the present invention (Example 1), and the test piece was sealed in a moisture-proof bag in a low-humidity (20 %RH) environment.

### <Examples 2 to 11>

Test pieces 6 to 15 for measuring protein (Examples 2 to 11) were produced in the same manner as in Example 1, except that in the test piece 1 for measuring protein produced in Example 1, the type and content of the hydroxy acid were changed as shown in Table 1, and the test pieces were sealed in a moisture-proof bag in a low-humidity (20 %RH) environment.

### <Comparative Examples 1 to 4>

Test pieces 1 to 4 for measuring protein (Comparative Examples 1 to 4) were produced in the same manner as in Example 1, except that in the test piece 1 for measuring protein produced in Example 1, the type and content of the hydroxy acid were changed as shown in Table 1, and the test pieces were sealed in a moisture-proof bag in a low-humidity (20 %RH) environment.

### <Evaluation of protein level>

The evaluation was performed using albumin, which is the most common protein contained in the biological specimen. In addition, the concentration for evaluation was set to 400 mg/100 mL, which is the upper limit of the quantifiable concentration range obtainable with a general protein assay test piece.

An inspection solution containing 400 mg/100 mL of human serum albumin (HSA) was prepared, the test pieces 1 to 15 for measuring protein produced in Comparative Examples 1 to 4 and Examples 1 to 11 were taken out of the moisture-proof bag, then 10 µL of the inspection solution was immediately spotted on the test piece, and the albumin level was measured using FUJI DRI-CHEM 7000 (manufactured by FUJIFILM Corporation) to determine ΔOD, which is a difference between an OD in a case where the protein is present and an OD in a case where the protein is not present. The measurement results were evaluated using an average value of three repeated measurements. The results of the evaluation are shown in Table 1.

**[Table 1]**

| Test piece for measuring protein | Hydroxy acid | Mol ratio (hydroxy acid/PV) | ΔOD | Note |
|---|---|---|---|---|
| 1 | Tartaric acid | 3.00 | 0.14 | Comparative Example 1 |
| 2 | Malic acid | 3.00 | 0.02 | Comparative Example 2 |
| 3 | Citric acid | 3.00 | 0.17 | Comparative Example 3 |
| 4 | Citric acid | 60.05 | 0.15 | Comparative Example 4 |
| 5 | Tartaric acid | 6.03 | 0.23 | Example 1 |
| 6 | Tartaric acid | 12.01 | 0.39 | Example 2 |
| 7 | Malic acid | 12.01 | 0.25 | Example 3 |
| 8 | Citric acid | 12.01 | 0.41 | Example 4 |
| 9 | Tartaric acid | 20.42 | 0.36 | Example 5 |
| 10 | Malic acid | 20.42 | 0.40 | Example 6 |
| 11 | Citric acid | 20.42 | 0.42 | Example 7 |
| 12 | Malic acid | 24.02 | 0.38 | Example 8 |
| 13 | Citric acid | 24.02 | 0.38 | Example 9 |
| 14 | Malic acid | 36.03 | 0.40 | Example 10 |
| 15 | Citric acid | 36.03 | 0.30 | Example 11 |

### <Evaluation results>

It was found that ΔOD was the largest in a case where the content of the hydroxy acid was 6.03 to 36.03 times that of PV in terms of molar ratio.

## Claims

1. A test piece for measuring protein, comprising:
a support; and
a reagent-holding layer provided on the support,
wherein the reagent-holding layer contains a coloring agent that changes color in a presence of protein, a molybdate, and a hydroxy acid having a hydroxy group and a carboxy group, and
a content of the hydroxy acid is 5 times or more and 40 times or less with respect to the coloring agent that changes color in the presence of protein in terms of molar ratio.

2. The test piece for measuring protein according to claim 1,
wherein the coloring agent that changes color in the presence of protein is pyrogallol red or pyrocatechol violet.

3. The test piece for measuring protein according to claim 1,
wherein the coloring agent that changes color in the presence of protein is pyrocatechol violet.

4. The test piece for measuring protein according to any one of claims 1 to 3,
wherein the hydroxy acid is a compound having two or more carboxy groups.

5. The test piece for measuring protein according to any one of claims 1 to 3,
wherein the hydroxy acid includes at least one of tartaric acid, citric acid, or malic acid.

6. The test piece for measuring protein according to any one of claims 1 to 3,
wherein the reagent-holding layer includes a water-absorbent layer and a spreading layer.

7. A method for measuring protein, comprising:
spotting a protein-containing sample on the test piece for measuring protein according to any one of claims 1 to 3; and
measuring color development in the reagent-holding layer.
